# EUROPÄISCHE PATENTANMELDUNG

(11) **EP 0 744 188 A2**
(43) Veröffentlichungstag der Anmeldung: **27.11.1996**
(21) Anmeldenummer: 96107625.4
(22) Anmeldetag: 14.05.1996
(51) Int. Cl.: A61M 31/00

(54) **Nasal-Applikator**

(30) Priorität: 26.05.1995 DE 19518810
(71) Anmelder: BAYER AG, 51368 Leverkusen (DE)
(72) Erfinder: Herold, Heiko, 41470 Neuss (DE); Wollenschläger, Axel, Dr., 51467 Bergisch Gladbach (DE); von Schuckmann, Alfred, 47627 Kevelaer (DE)

(57) **Zusammenfassung**

Nasal-Applikatoren sind ein wichtiges Hilfsmittel, um pulverförmige, pharmakologisch wirksame Arzneistoffe in den Nasen/Rachen-Raum eines Patienten einzubringen. Der hier beschriebene Nasal-Applikator geht aus vor einer handbetätigten, mit einem Zuströmkanal (8) verbundenen Druckluftquelle zur Erzeugung eines Druckluftstoßes, einem Vorratsbehälter (3) für den Arzneistoff und einer Dosiereinrichtung zur Bereitstellung einer abgeteilten Arzneistoffmenge, die von dem Druckluftstoß erfaßt und durch einen Abströmkanal (9) in die Nase transportiert wird. Dabei besteht die Dosiereinrichtung aus einer Dosiertrommel (5), bei der der Vorratsbehälter (3) für den Arzneistoff durch den Innenraum der Dosiertrommel (5) gebildet wird. Weiterhin ist der Zuströmkanal (8) mit dem einen Ende eines Dosierkanals (7) und der Abströmkanal (9) mit dem anderen Ende des Dosierkanals (7) verbunden. Die in einer Abteilkammer (13) der Dosiertrommel (5) bereitgestellte Arzneistoffmenge ragt in der Weise in den Dosierkanal (7) hinein, daß sie von dem Druckluftstrom vollständig erfaßt und in die Nasenkammern transportiert wird.

## Beschreibung

Die Erfindung betrifft eine Vorrichtung zur Applikation eines pulverförmigen Arzneimittels in die Nase.

Pulverförmige Arzneimittel dienen zur Behandlung entzündlicher und allergischer Erkrankungen der Nasenschleimhäute.

Darüberhinaus kommt eine nasale Applikation auch für bestimmte systemisch zu verabreichende Wirkstoffe (z.B. kurzkettige Peptide) in Frage. Hierdurch lassen sich in manchen Fällen die für die Patienten unangenehmen Injektionen vermeiden.

Einige der für die nasale Applikation von Pulvern entwickelten Geräte enthalten den Wirkstoff in abgeteilter Form in Hartgelatinekapseln, die unmittelbar vor der Inhalation in das Inhalationsgerät geladen und geöffnet werden müssen.

Dieser Ladevorgang ist umständlich und für ältere Menschen mit rheumatischen Beschwerden oder schlechtem Sehvermögen nicht befriedigend möglich.

Zur Überwindung dieses Nachteiles wurde ein selbstdosierendes Gerät entwickelt (Offenlegungsschrift 2 529 522), bei dem der umständliche Ladevorgang entfällt. Nachteilig bei diesem Gerät ist jedoch, daß der Patient durch aktive Inhalation das Pulver aufnehmen muß. Dies ist insbesondere bei geschwollenen Schleimhäuten nicht befriedigend möglich.

Um den Wirkstoff aktiv in die Nase hineinzublasen, wurden auch Geräte entwickelt, bei denen der Wirkstoff in gelöster oder suspendierter Form in einem druckverflüssigten Treibgas vorliegt. Bei Betätigung des Dosierventils wird eine abgeteilte Menge der Wirkstoffsuspension freigesetzt. Das unter eigenem Dampfdruck stehende Treibmittel verdampft unmittelbar, dispergiert den Wirkstoff und bläst in die Nasenöffnung. Ein Nachteil dieser Applikationsmethode besteht in der Verwendung von druckverflüssigten Treibmitteln, welche zusätzlich vom Körper des Patienten aufgenommen werden müssen. Häufig sind weitere Hilfsstoffe wie z.B. Ventilschmiermittel, Antiflockungsmittel etc. den Formulierungen beizugeben. Schließlich werden auch die im pharmazeutischen Bereich eingesetzten Treibmittel wegen nachteiliger Beeinflussung der Umwelt (z.B. Beitrag zur Erwärmung der Erdatmosphäre und/oder Zerstörung der Ozonschicht) angefeindet.

Aufgabenstellung war, ein bedienerfreundliches, selbstdosierendes Inhalationsgerät zu entwickeln, das gegebenenfalls ohne die Verwendung von druckverflüssigten Treibmitteln auskommt und den Wirkstoff aktiv in die Nasenhöhle bläst.

Gelöst ist diese Aufgabe durch die im Anspruch 1 angegebene Erfindung.

Die daran anschließenden Ansprüche geben weitere vorteilhafte Ausgestaltungen der erfindungsgemäßen Vorrichtung wieder.

Zufolge solcher Ausgestaltung ist eine gattungsgemäße Vorrichtung vereinfachten Aufbaus und erhöhten Gebrauchs- und Sicherheitswerts erzielt. Der mechanische Aufwand ist stark reduziert. Die Menge wird in Ruhe in die Ausgabebereitschaftsstellung gebracht. Der Benutzer kann sich auf das Einbringen des Wirkstoffes konzentrieren. Konkret ist der Applikator erfindungsgemäß so ausgebildet, daß die Dosiereinrichtung aus einer Dosiertrommel besteht, bei der der Vorratsbehälter für den Arzneistoff durch den Innenraum der Dosiertrommel gebildet wird, daß der Zuströmkanal mit dem einen Ende des Dosierkanals und der Abströmkanal mit dem anderen Ende des Dosierkanals verbunden ist und daß die in einem Abteildurchbruch der Dosiertrommel bereitgestellte Arzneistoffmenge in den Dosierkanal hineinragt. Der Innenraum der Dosiertrommel ist nun für die Vorratsbildung genutzt. Ihr Inhalt wird in Bewegung gehalten. Er gelangt auf kürzestem Wege in die Ausgabereitschaftsstellung. Dort erfolgt eine restfreie Austragung. Es können aufgrund der geschilderten Ausrichtung und Aneinanderreihung der Funktionsbereiche keine Partikel zurückfallen. Die Druckluft trifft eingangs auf die Arzneistoffmenge und strömt in die exakt positionierte und abgeteilte, exponierte Anhäufung des Arzneistoffes. Die kuppelförmig dargebotene Zone wird rasch abgetragen, verwirbelt bzw. dispergiert und bei Betätigung der Druckluftquelle an den Zielort verbracht. Der Arzneistoff wird gegen die Wirkung der Schwerkraft geblasen. Fehlhaltungen sind praktisch ausgeschlossen, da die Lage der Dosiertrommel die korrekte Handhabung suggeriert und der am anderen, oberen Ende liegende Abströmkanal mit der Applikatordüse als Übergabebrücke dem Benutzer unschwer auffällt. Außerdem kann sich kein Potential für eine Überdosierung bilden; eine nicht applizierte Pulvermenge verschwindet in Richtung Vorrat. Sie wird nicht durch den nächsten Schaltschritt ergänzt. Was nun die Ausbildung der Dosiertrommel betrifft, so ist diese in der Weise ausgeführt, daß sie eine mit Abteildurchbrüchen ausgestattete Drehhülse besitzt. Die in gleicher Winkelverteilung angeordneten Abteildurchbrüche tauchen aufgrund der mischtrommelartigen Drehung der Dosiertrommel in den Pulvervorrat ein, ohne daß es zu Pressungen etc. kommt. Die Dosierung ist einschließlich der vorletzten Abteilung und gegebenenfalls bis zur letzten Abteilung volumengerecht. Es kommt auch nicht zu einer Blockbildung des Arzneistoffes aufgrund der Nutzung der Trommelbewegung. Weiter ist vorgesehen, daß die Drehhülse von der Mantelwand eines Topfes gebildet ist, dessen Boden einen Drehknopf aufweist. In Bezug auf die Drehlagerung der Dosiertrommel und die Erzielung stets gleicher Arzneistoff-Inhaliermengen erweist es sich als vorteilhaft, daß die von einer Außenwand umfaßte Drehhülse über einen Teilwinkelbereich von einem inneren Wandabschnitt unterfangen ist, welcher den Boden der von jeweils einer der Durchbrechungen gebildeten Abteilkammer bildet. In diesem Zusammenhang ist es zudem vorteilhaft, daß die Querwände der Durchbrechungen zum Zentrum der Dosiertrommel hin trapezförmig und keilförmig verlaufen. Dies führt bezüglich der Querwände zu einem sich V-scheitelseitig, also nach oben gerichteten, konvex gebogenen, trapezförmigen Trog als Abteilkammer. Eine weitere vor allem herstellungstechnisch günstige Verbesserung besteht darin, daß alle Luftkanäle in einem von zwei Gehäuseschalen umschließbaren Träger eingearbeitet sind, in dem auch die Dosiertrommel gelagert ist, wobei die Gehäuseschalen die Abdeckung für die Luftkanäle bilden. Außerdem kann vorgesehen werden, daß der Träger einen Standfuß der als Standgerät gestalteten Vorrichtung formt, dessen Schulter einen Aufsteckbegrenzungsanschlag für die Schutzkappe bildet.

Mit der Erfindung werden folgende weitere Vorteile erzielt:
- Der Applikator eignet sich auch für Arzneistoff-Formulierungen, die schlecht fließfähig sind oder zum Anbacken neigen. Aufgrund der gleichzeitigen Homogenisierung bei jedem Dosiervorgang werden evtl. vorhandene Pulverbrücken aufgebrochen und aufgelockert.
- Ferner hat sich herausgestellt, daß sich bei geringen Abweichungen von der vorgeschriebenen Gebrauchslage die Dosiergenauigkeit nicht merklich verschlechtert; d.h. geringe Abweichungen von der Gebrauchslage sind im Hinblick auf die Dosiergenauigkeit unkritisch. Nach der Dosierung, d.h. nach Betätigung der Dosiertrommel, kann die Inhalation in jeder beliebigen Lage erfolgen.
- Aufgrund der spritzgußfreundlichen Konstruktion und aufgrund der geringen Zahl von erforderlichen Bauteilen kann der Applikator kostengünstig und wirtschaftlich in großen Stückzahlen hergestellt werden.

Der Gegenstand der Erfindung ist nachstehend anhand eines zeichnerisch veranschaulichten Ausführungsbeispieles näher erläutert. Es zeigen:
- Fig. 1: eine Draufsicht des Nasal-Applikators,
- Fig. 2: eine Seitenansicht,
- Fig. 3: einen Detailausschnitt der Dosiervorrichtung,
- Fig. 4: eine Draufsicht einer modifizierten Ausführung des Nasal-Applikators.

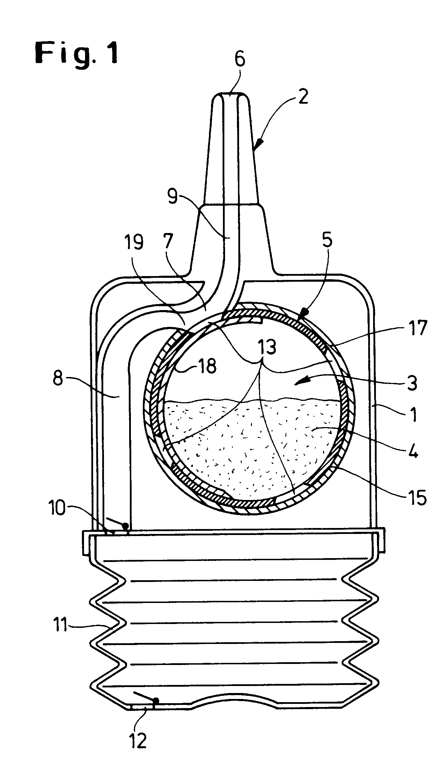

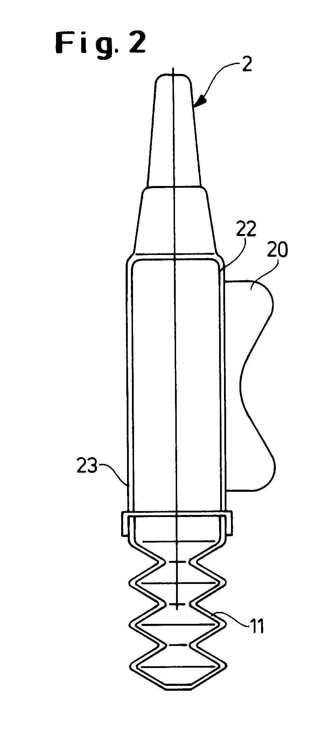

Die als Taschengerät ausgebildete Vorrichtung, nachstehend Applikator genannt, besitzt gemäß Fig. 1 und 2 ein lang-rechteckiges, im Taschenformat ausgebildetes, flaches Gehäuse 1. Dessen Kopfbereich enthält einen sogenannten Nasal-Applikator 2. Bei der nachfolgenden Beschreibung wird vorausgesetzt, daß sich der Applikator in einer Vertikalebene befindet, die in den Figuren mit der Zeichenebene übereinstimmt.

Der Applikator enthält einen Vorratsbehälter 3, der mit pulverförmigem Arzneistoff 4 (Formulierung) gefüllt ist.

Der Vorratsbehälter 3 ist vom Innenraum einer Dosiertrommel 5 gebildet. Die Dosiertrommel 5 ist drehbar im Gehäuse 1 gelagert und von außen her unmittelbar handbetätigbar. Sie befindet sich im Fußbereich des Applikators und nutzt volumenmäßig nahezu die gesamte Breite des Gehäuses 1.

Der Applikator wird beim Gebrauch so gehalten, daß sich das Dosierteil unten und der Adapter 2 mit der Applikatoröffnung 6 oben befindet (Gebrauchsstellung). Diese Stellung entspricht auch der in Fig. 1 und Fig. 2 gewählten Darstellung.

Dosiertrommel 5 und Adapter 2 liegen also in der Gebrauchsstellung des Applikators praktisch vertikal übereinander. Die pulverförmigen Partikel werden somit bei der Applikation von unten nach oben gefördert.

Die grundsätzliche Funktion der Dosiertrommel 5 besteht darin, daß bei einem Dosiervorgang durch Drehung der Trommel eine definierte Arzneistoffmenge aus dem Vorratsbehälter 3 in einen Dosierkanal 7 gebracht wird und von dort mittels eines impulsartigen Luftstroms durch die Applikatoröffnung 6 in den Nasenraum gefördert wird. Zu diesem Zweck ist der Dosierkanal 7 einerseits mit einem Zuströmkanal 8 und andererseits mit einem Abströmkanal 9 verbunden, der zur Applikatoröffnung 6 im Nasaladapter 2 führt. Der Zuströmkanal 8 ist über ein Auslaßventil 10 mit einem Faltenbalg 11 verbunden. Außerdem ist an dem Faltenbalg 11 ein Einlaßventil 12 angebracht. Bei Betätigung des Faltenbalges 11 (Zusammendrücken) wird ein Luftstoß erzeugt, der über den Zustromkanal 8 in den Dosierkanal 7 strömt und dort die bereitgestellte Arzneistoffmenge durch den anschließenden Abströmkanal 9 austrägt und durch die Applikatoröffnung 6 in die Nase des Patienten befördert. Im Dosierkanal 7 wird also die exakt dosierte, aus dem Vorratsbehälter 3 entnommene Pulvermenge dem Druckluftstoß zur Austragung dargeboten.

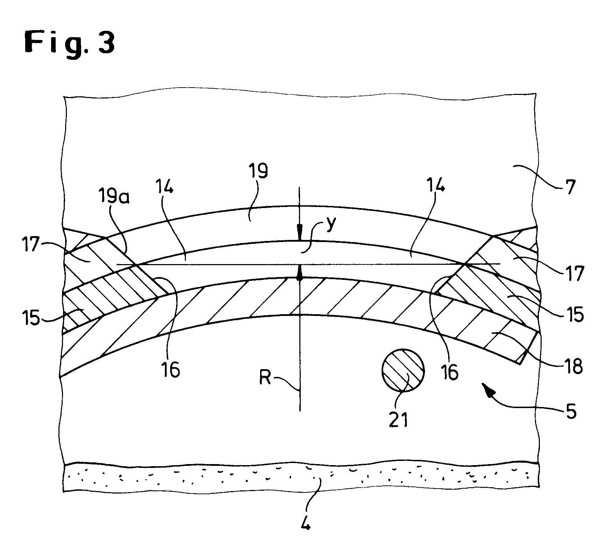

Die Dosiertrommel 5 weist zu diesem Zweck kanalseitig offene Abteilkammern 13 auf. Aufgrund der kreisförmigen Wölbung der Dosiertrommel 5 wird der abgeteilte Arzneistoff (Formulierung) im Dosierkanal 7 als vorgewölbte Schicht bereitgestellt. Die daraus resultierende Arzneistoff-Kuppel läßt sich besonders strömungsgünstig ab- bzw. ausräumen. Die konvexe Wölbung der Arzneistoff-Kuppel geht besonders deutlich aus Fig. 3 hervor. Dort ist die aus der Wölbung der Abteilkammer 13 resultierende Überhöhung y (angegeben in der Sehnenmitte) der auf das Zentrum der Dosiertrommel 5 gerichteten Radialen R gut erkennbar. Das Zentrum liegt auf der geometrischen Drehachse der Dosiertrommel 5.

Wie aus den Fig. 1 und insbesondere 3 ersichtlich ist, wird eine Abteilkammer 13 durch einen Durchbruch 14 in einer Drehhülse 15 der als Topf gestalteten Dosiertrommel 5 gebildet. Gemäß Fig. 1 sind vier Durchbrüche 14 bzw. Abteilkammern 13 gleichmäßig über den Umfang der Drehhülse 15 verteilt. Die Durchbrüche 14 sind durch Seitenwände 16 begrenzt, die derart abgeschrägt sind, daß sich die Durchbrüche 14 zum Zentrum der Dosiertrommel 5 hin verjüngen. Der Schrägungswinkel liegt bei 45° zur kürzesten Verbindung zwischen Außen- und Innenfläche der Dosiertrommel-Wandung. Die Querwände 16 verleihen der Abteilkammer 13 die Form eines trapezförmigen, nach außen öffnenden, gebogenen Troges von deutlich größerer Länge als Breite.

Die Drehhülse 15 ist drehbar zwischen einer Ringwand 17 und einem ebenfalls ringförmigen, sich über einen Bogenwinkel von ca. 180° erstreckenden Wandungsabschnitt 18 gelagert. Der etwa halbkreisförmige, stegförmige Wandungsabschnitt 18 ist dabei so angeordnet, daß er einerseits den Bodenbereich des Dosierkanals 7 bildet und zu keinem Zeitpunkt eine durchgehende Verbindung zwischen den Luftkanälen 8 und 9 und dem Vorratsbehälter 3 besteht und andererseits am unteren Ende die Fußzone der Drehhülse 15 freibleibt (s. Fig. 1). Die Ringwand 17 und der Wandungsabschnitt 18 sind an dem Gehäuse 1 angeformt. Die Lagerung der Drehhülse kann durch einen etwa 1 bis 2 mm hohen Lagerkragen verbessert werden, der sich in Verlängerung des besagten Wandungsabschnitt 18 über den verbleibenden Umfangswinkel erstreckt und ebenfalls am Gehäuse 1 angeformt ist. Auf diese Weise wird die Mantelfläche der Drehhülse 15 in dem Ringspalt zwischen der Ringwand 17 und dem Wandungsabschnitt 18 einschließlich des erwähnten Lagerkragens exakt drehbar geführt. Die Ringwand 17 weist gegenüber dem Dosierkanal 7 ein Eintrittsfenster 19 auf. Die Seitenflächen 19a des Eintrittsfensters 19 sind in der Weise abgeschrägt, daß sie mit den Seitenflächen 16 der Abteilkammer 13 fluchten, wenn sich die Abteilkammer 13 genau unter dem Eintrittsfenster 19 befindet. Solange sich eine Abteilkammer 13 innerhalb des Winkelbereichs des Wandungsabschnitts 18 befindet, fungiert die Außenfläche des Wandungsabschnitts 18 als Boden für die trogförmige Abteilkammer 13. Aus diesem Grund ist die Höhe des stegförmigen Wandungsabschnitts 18 etwas größer bemessen als die Breite des Durchbruchs 14. Bei einer weiteren Drehung der Drehhülse 15 im Uhrzeigersinn, d.h. nachdem sich die Abteilkammer 13 unter dem Eintrittsfenster 19 hinweg bis über das rechte obere Ende des Wandungsabschnitts 18 hinausgeschoben hat, ist der Boden der Abteilkammer 13 offen, so daß sie wieder mit dem Vorratsbehälter 3 in Verbindung steht.

Während der besagte Wandungsabschnitt 18 im Bereich des Dosierkanals 7 mit seiner Außenfläche als Boden fungiert, deckt er im Bereich der ansteigenden, im Uhrzeigersinn vorgelagerten Abteilkammer die aus dem Pulvervorrat genommene Pulvermenge des nächstfolgenden Durchbruchs 14 von innen her gegen den Vorrat ab. In Fig. 1 befindet sich diese Abteilkammer gerade in einer "8 Uhr-Stellung", während die zum Inhalieren bereitstehende Abteilkammer in einer "11 Uhr-Position" steht. Das Laden einer Abteilkammer 13 erfolgt in der Weise, daß sich der dazugehörige Durchbruch 14 beim Weiterdrehen der Drehhülse 15 unter dem Pulvervorrat 4 hindurchschiebt (in Fig. 1 etwa in einer Zone von "4 Uhr" bis "6 Uhr"), wobei sich diese Abteilkammer 13 mit dem Pulver füllt. Bei der weiteren Drehung der Drehhülse 15 gelangt diese Abteilkammer in den ansteigenden Bereich zwischen "6 Uhr" und "9 Uhr". In diesem Bereich erfolgt die Mengensicherung der Inhalationsdosis in der Abteilkammer durch den absperrend wirkenden Wandungsabschnitt 18, dessen unteres Ende als Abstreifer fungiert, so daß ein eventuell vorhandener einwärts gerichteter Pulverüberstand abgeschabt wird. Auf diese Weise wird eine genaue Dosis mit reproduzierbarer Pulverdichte erreicht.

Der Wandungsabschnitt 18 läßt ca. 180° zum Innenraum des Vorratsbehälters 3 hin offen. Die Drehhülse 15 wirkt wie ein Drehschieber. Wird die dosierte Pulvermenge nicht entnommen, gelangt sie beim Weiterdrehen der Dosiertrommel 5 automatisch wieder in den Vorrat, also den Vorratsbehälter 3, zurück. Eine Mengenaddition ist nicht möglich. Dieser wesentliche Vorteil ergibt sich aus der Vertikalorientierung der Funktionsorgane.

Die Mantelwandung der Drehhülse 15 der als Topf gestalteten Dosiertrommel 5 ist außenseitig mit einem bequem zugänglichen Drehknopf 20 versehen (siehe insbesondere Fig. 2).

Am Topfboden sind parallel zur Drehachse der Dosiertrommel 5 gleichmäßig über den Umfang verteilt mehrere Auflockerungsfinger 21 angebracht, die in den Pulvervorrat hineinragen und deren radialer Abstand vom Zentrum so gewählt ist, daß sie bei der Drehung den Rücken des Wandungsabschnittes 18 abstreifen, so daß sich dort kein Pulver festsetzen kann. Im Uhrzeigersinn gesehen, ist gemäß Fig. 1 unmittelbar hinter jedem Durchbruch 14 (voreilend) ein Auflockerungsfinger 21 angeordnet. Alternativ oder auch zusätzlich können die Auflockerungsfinger ortsfest am Gehäuse 1 angebracht sein, wobei ebenfalls die Trommelbewegung zur Auflockerung des Pulvers genutzt wird.

Das Applikatorgehäuse 1 besteht aus zwei im Querschnitt U-förmigen Gehäuseschalen 22 und 23. Die beiden Schalen 22 und 23 umschließen sandwich-artig einen Träger (nicht gezeichnet) als Kernteil, in dem als wesentliche Bestandteile der Zuströmkanal 8, der Dosierkanal 7 und der Abströmkanal 9, sowie eine kreisrunde Aussparung für die Halterung der Dosiertrommel 5 untergebracht sind.

Die Luftkanäle 7, 8, 9 sind als rinnenartige Vertiefungen mit annähernd rechteckigem Querschnitt ausgebildet und werden durch die Gehäuseschalen 22 und 23 abgeschlossen bzw. abgedeckt. Diese Konstruktion gewährleistet auch dann eine ausreichende mechanische Stabilität, wenn der Träger aus Gründen der Gewichtsersparnis relativ dünnwandig (z.B. ca. 5 mm) gestaltet wird.

Es kann eine klemmtechnische Befestigungsweise der Gehäuseteile 22, 23 zugrunde liegen; eine Verklebung oder thermische Anbindung ist ebenfalls denkbar, wenn nicht zu Reinigungszwecken respektive Nachfüllzwecken die völlige bzw. teilweise Zerlegbarkeit des Gehäuses 1 angestrebt wird. In diesem Falle sind dann die üblichen klassischen Befestigungsmittel, wie Verschraubung etc., vorzuziehen.

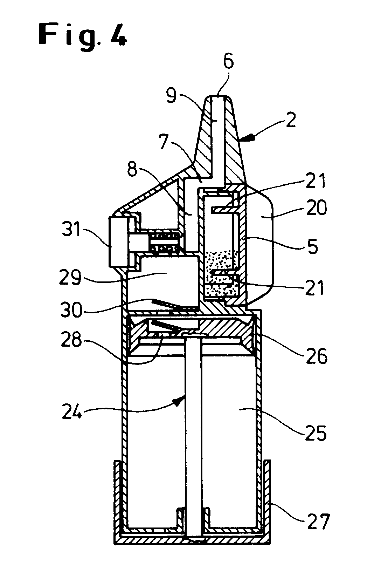

Bei der modifizierten Ausführung des Applikators gemäß Fig. 4 besteht die Druckluftquelle aus einer handbetätigten Kolbenpumpe 24, die einen in einer Pumpkammer 25 verschiebbaren Pumpkolben 26 mit einem Handgriff 27 und einem Ventil 28 aufweist. Die Pumpkammer 25 ist hier über eine Kompressionskammer 29 mit dem Zuströmkanal 8 verbunden. Am Eintritt in die Kompressionskammer 29 (von der Pumpkammer her gesehen) befindet sich ein Einlaßventil 30. Durch Betätigung eines mittels einer Feder vorgespannten Auslöseknopfes 31 kann die Kompressionskammer 29 mit dem Zuströmkanal 8 verbunden werden.

Nachfolgend soll noch einmal die Funktion der Applikatoren gemäß Fig. 1 bis 4 beschrieben werden.

Gemäß Fig. 1 befindet sich der pulverförmige Arzneistoff innerhalb der Drehhülse 15 und gelangt dort in die unteren Abteilkammern 13. Beim Drehen der Drehhülse 15 gelangt nun die gefüllte Abteilkammer 13 zunächst zwischen Ringwand 17 und Wandungsabschnitt 18. Ein Entleeren der Abteilkammer 13 kann nun erst erfolgen, wenn sie in den Bereich des Dosierkanals 7 gedreht wird, da hier die Ringwand 17 unterbrochen ist. Die Vorrichtung wird vom Benutzer mit dem Nasaladapter 2 in die Nase eingeführt. Durch Zusammenpressen des Faltenbalgs 11 wird nun über das Druckventil 10 ein Luftvolumen in den Dosierkanal 7 gepreßt. Durch den dabei entstehenden Luftstoß wird die bereitgestellte Abteilkammer 13 entleert und der Kammerinhalt mit dem Luftstrom in die Nase geblasen. Beim anschließenden Entspannen des Faltenbalges 11 schließt sich das Druckventil 10 (Auslaßventil), gleichzeitig öffnet sich das Einlaßventil 12, um ein Einströmen von Luft in den Faltenbalg 11 zu ermöglichen.

Gemäß Fig. 4 wird der pulverförmige Arzneistoff wiederum in der Drehhülse 15 bereitgestellt, wobei sich die unten liegenden Abteilkammern 13 mit dem Pulver füllen. Dabei wird der pulverförmige Arzneistoff beim Drehen der Drehhülse 15 durch in der Hülse angebrachte Lockerungsstifte 21 aufgelockert, um ein gutes Fließen zu erreichen. Durch Hochdrücken des Pumpkolbens 26 mit dem Handgriff 27 wird der Luftinhalt der Pumpkammer 25 über das Druckventil 28 und das Einlaßventil 30 in die Kompressionskammer 29 verschoben. Hat man nun vorher durch Betätigung der Dosiertrommel 5 eine mit dem Arzneistoff gefüllte Abteilkammer 13 im Dosierkanal 7 bereitgestellt, so wird durch Drücken des Auslöseknopfes 31 die in der Kompressionskammer verdichtete Luft freigegeben, so daß ein impulsartiger Luftstrom (Luftstoß) den nachfolgenden Zuströmkanal 8 mit hoher Geschwindigkeit durchströmt, die im Dosierkanal bereitgestellte, pulverförmige Arzneistoffmenge vollständig mitreißt und durch den Abströmkanal 9 und die Applikatoröffnung 6 in die Nase des Patienten transportiert.

## Patentansprüche

1. Nasal-Applikator zum Einbringen eines pulverförmigen, pharmakologisch wirksamen Arzneistoffs in die Nase, bestehend aus einer handbetätigten, mit einem Zuströmkanal (8) verbundenen Druckluftquelle zur Erzeugung eines Druckluftstoßes, einem Vorratsbehälter (3) für den Arzneistoff; einer Dosiereinrichtung zur Bereitstellung einer abgeteilten Arzneistoffmenge, die von dem Druckluftstoß erfaßt und durch einen Abströmkanal (9) in die Nase transportiert wird, dadurch gekennzeichnet, daß die Dosiereinrichtung aus einer Dosiertrommel (5) besteht, bei der der Vorratsbehälter (3) für den Arzneistoff durch den Innenraum der Dosiertrommel (5) gebildet wird, daß der Zuströmkanal (8) mit dem einen Ende eines Dosierkanals (7) und der Abströmkanal (9) mit dem anderen Ende des Dosierkanals (7) verbunden ist und daß die in einer Abteilkammer (13) der Dosiertrommel (5) bereitgestellte Arzneistoffmenge in den Dosierkanal (7) hineinragt.

2. Nasal-Applikator nach Anspruch 1, dadurch gekennzeichnet, daß die Dosiertrommel (5) eine Drehhülse (15) aufweist, an deren Umfang die Abteilkammern (13) angeordnet sind.

3. Nasal-Applikator nach Anspruch 2, dadurch gekennzeichnet, daß die Drehhülse (15) von der Mantelwand eines Topfes gebildet ist, an dessen Boden ein Drehknopf (20) angeordnet ist.

4. Nasal-Applikator nach den Ansprüchen 2 und 3, dadurch gekennzeichnet, daß die Drehhülse (15) von einer Ringwand (17) umfaßt ist und über einen Teilwinkelbereich von einem inneren Wandabschnitt (18) unterfangen ist, welcher den Boden der von jeweils einem der Druchbrüche (14) gebildeten Abteilkammer (13) bildet.

5. Nasal-Applikator nach Anspruch 4, dadurch gekennzeichnet, daß die Querwände (16) der Durchbrüche (14) trapezförmig und keilförmig in Richtung zum Zentrum der Dosiertrommel (5) hin verlaufen.

6. Nasal-Applikator nach den Ansprüchen 2 bis 5, dadurch gekennzeichnet, daß die Kanäle (7, 8, 9) in einem von zwei Gehäuseschalen (22, 23) umschließbaren Träger untergebracht sind, in dem auch die Dosiertrommel (5) gelagert ist, wobei die Gehäuseschalen (22, 23) die Abdeckung für die Kanäle (7, 8, 9) bilden.

7. Nasal-Applikator nach den Ansprüchen 1 bis 6, dadurch gekennzeichnet, daß die Druckluftquelle aus einem Faltenbalg (11) besteht.

8. Nasal-Applikator nach Ansprüchen 1 bis 7, dadurch gekennzeichnet, daß zwischen Druckluftquelle und Zuströmkanal (8) eine Kompressionskammer (29) und zwischen der Kompressionskammer (29) und dem Zuströmkanal (8) ein Auslöseknopf (31) angeordnet ist, bei dessen Betätigung der in der Kompressionskammer (29) gespeicherte Luftvorrat freigegeben wird.
